# EUROPEAN PATENT APPLICATION

(11) **EP 2 246 319 A1**
(43) Date of publication of application: **03.11.2010**
(21) Application number: 09158953.1
(22) Date of filing: 28.04.2009
(51) Int. Cl.: C07C 51/43, C07C 57/15, C12P 7/46

(54) **Fumaric acid crystallization process**

(71) Applicant: DSM IP Assets B.V., 6411 TE Heerlen (NL)
(72) Inventor: Graaf, Van De, Maarten Job, 2722 XB, ZOETERMEER (NL); Ganendra, Giovanni, 2611 BJ, DELFT (NL)
(74) Representative: Cazemier, Anne Engeline

(57) **Abstract**

Process for the crystallization of fumaric acid, comprising crystallizing fumaric acid in an aqueous solution having a pH of between 0.5 to 3.5, wherein the aqueous solution comprises microbial cells at a concentration of at least 0.5 to 20 g dry weight / I, wherein fumaric acid crystals are formed.

## Description

### Field of the invention

The present invention relates to a process for the crystallization of fumaric acid.

### Background of the invention

Fumaric acid is a widely known compound, which finds use in several food, medical and cosmetic applications. Fumaric acid is predominantly produced by petrochemical routes, eg. by isomerization of maleic acid. Since petrochemical routes are considered harmful to the environment, it is desired to produce fumaric acid via a biological route. *Rhizopus* is a prospective fumaric acid producer, which can produce fumaric acid by fermentation. In a typical fumaric acid fermentation with *Rhizopus oryzae* the presence of a neutralizing agent is required not only to neutralize the acid during fermentation, but also to remove fumaric acid produced from the fermentation broth. Recovery of calcium fumarate and regeneration of free acid from fumarate are complicated, tedious, and expensive. Therefore there is a need for an economically attractive fumaric acid fermentation and recovery process.

The aim of the present invention is a process for the recovery of fumaric acid at a sufficient high purity in which no or a limited amount of neutralizing agent needs to be added.

### Detailed description of the invention

The aim is achieved according to the present invention with a process for the crystallization of fumaric acid, comprising crystallizing fumaric acid in an aqueous solution having a pH of between 0.5 to 3.5, wherein the aqueous solution comprises microbial cells at a concentration of at least 0.5 to 20 g / l, wherein fumaric acid crystals are formed.

Surprisingly, it was found that the fumaric acid crystals crystallized in the process according to the present invention were obtained in a high purity. A high purity is herein defined as fumaric acid crystals comprising at least 80% w/w fumaric acid, preferably at least 82, 84, 86, 88%, more preferably at least 90, 92%, more preferably at least 95% w/w fumaric acid.

lt was also surprisingly found in the process according to the present invention that no coagulation of fumaric acid crystals with biomass occurred.

The aqueous solution in the process according to the present invention preferably is a fermentation broth. Preferably, the process according to the present invention comprises fermenting a microbial cell at a pH of between 0.5 and 3.5 in a fermentation broth and producing fumaric acid. Preferably, the process according to the present invention comprises the concomitant producing of fumaric acid by fermenting a microbial cell at a pH of between 0.5 and 3.5, and crystallizing of fumaric acid to form fumaric acid crystals in a fermentation broth. It was found advantageous to crystallize fumaric acid during the production of fumaric acid, since this lowers the concentration of fumaric acid in the fermentation broth.

In one embodiment, the pH in the process according to the present invention preferably is below the lowest pKa of fumaric acid, preferably between 1 and 3.2, preferably between 2 and 3. It was found that at pH values below the lowest pKa of fumaric acid, fumaric acid crystals were obtained at a high purity.

In one embodiment, the aqueous solution comprises microbial cells at a concentration of at least 0.5 to 20 g dry weight / I, preferably between 1 and 15, preferably between 2 and 10 g dry weight / I.

A microbial cell in the process according to the present invention preferably is a non-filamentous fungus. Preferably, the microbial cell is a *Saccharomyces, Pichia, Kluyveromyces, Yarrowia, Candida, Hansenula, Humicola, Torulaspora, Trichosporon, Brettanomyces, Rhizopus, Zygosaccharomyces, Pachysolen* or *Yamadazyma.*
Preferably the cell is a *Saccharomyces* sp., preferably a *Saccharomyces cerevisieae.*

In a preferred embodiment the process of the invention further comprises separating the fumaric acid crystals from the aqueous solution.

Separating the fumaric acid crystals from the aqueous solution may be performed by centrifugation or sedimentation. Preferably, the separating comprises centrifugating the aqueous solution. Preferably, centrifugating the aqueous solution is performed at low centrifugation intensity. Centrifugation intensity is herein defined as the product of G-force (g) and centrifugation residence time (min). The centrifugation residence time is herein defined as the time during which the aqueous solution is placed in the centrifugal field. A low centrifugation intensity as used herein is an intensity of between 0.1 x g.min and 1000 x g.min, preferably between 0.2 x g.min and 900 x g.min, more preferably between 0.5 x g.min and 800 x g.min. Surprisingly, it was found that at the preferred centrifugation intensities of the invention fumaric acid crystals were obtained with a fumaric acid content of at least 90% w/w dry weight of fumaric acid crystals, preferably at least 92, 94 or 96 % w/w dry weight of fumaric acid crystals.

In another embodiment, the fumaric acid crystals formed in the process according to the present invention have sufficiently large size. A sufficiently large size is used herein to indicate fumaric acid crystals with a diameter of more than 10 micrometres, preferably more than 50 micrometres, more preferably more than 100 micrometres and usually below 1000 micrometres, preferably below 800 micrometres. It was found that a sufficiently large size of fumaric acid crystals allowed easy separation of fumaric acid crystals and microbial cells in the process according to the present invention.

In one embodiment, the process for crystallizing fumaric acid according to the present invention further comprises washing the fumaric acid crystals, to obtain purified fumaric acid crystals.

Washing fumaric acid crystals may be performed by any suitable method known in the art, for instance dissolving the crystals in methanol or a mixture of methanol/water, filtering the methanol solution comprising dissolved fumaric acid, and crystallizing the dissolved fumaric acid to obtain fumaric acid crystals, for instance by evaporation.

In another aspect the present invention relates to fumaric acid crystals comprising 80% to 99.5 % w/w dry weight of fumaric acid and a protein content of between 0.1 and 15 g protein / kg fumaric acid. Surprisingly, it was found that fumaric acid crystals according to the present invention were obtainable by the process according to the present invention. Preferably, the fumaric acid crystals comprise 82% to 99.6% w/w dry weight of fumaric acid, preferably between 84% and 99.8% w/w dry weight of fumaric acid. The protein content of fumaric acid crystals of the invention is preferably between 0.2 and 10 g protein / kg fumaric acid, preferably between 0.5 and 8 g protein / kg fumaric acid.

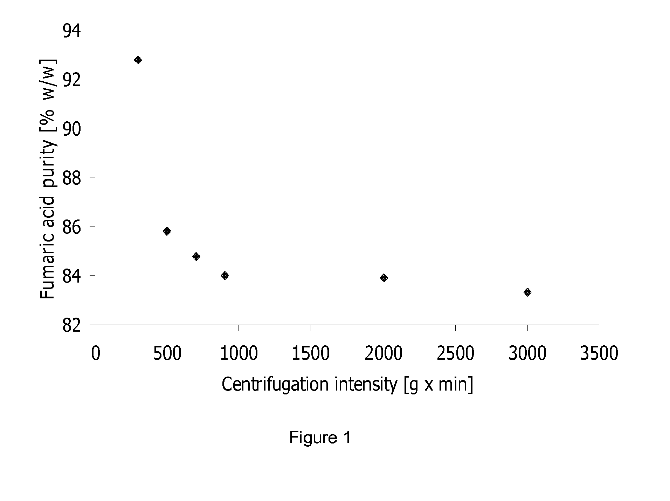

**Figure 1****:** Fumaric acid crystal content analyzed after separation of crystals from a slurry containing biomass and soluble organic and inorganic compounds. Separation was performed at various G-forces for a period of 1 minute.

The following examples are for illustrative purposes only and are not to be construed as limiting the invention.

### EXAMPLES

### Example 1

### Production of crude fumaric acid crystals in

A synthetic medium (see Table 1) was made in a 500 ml demineralized water according to the composition in Table 1.

**Table 1. Composition synthetic fumaric acid medium**

| Compound | Amount | Dimension¹ |
|---|---|---|
| | | |
| Fumaric acid | 45 | g/kg |
| Ammonium sulphate | 0.5 | g/kg |
| Magnesium sulphate (7aq) | 0.05 | g/kg |
| Succinic acid | 5 | g/kg |
| Ethanol | 5 | g/kg |
| Glycerol | 5 | g/kg |
| Pyruvic acid | 5 | g/kg |
| Acetic acid | 1 | g/kg |
| Malic acid | 5 | g/kg |
| Maltose | 5 | g/kg |

| | | |
|---|---|---|
| ¹ Gram per kilogram medium. | | |

Caustic soda (NaOH) was added until the fumaric acid was completely dissolved (clear solution at 30 °C).

Dried yeast (Fermipan, dried *Saccharomyces cerevisiae*) was added to the synthetic fumaric acid medium (a final concentration of 5 g/l), under low stirring rate (220 rpm)

The synthetic medium which contained yeast, was added to a vessel (0.5 I fermenter) and stirred slowly (220 rpm).

A 0.8 M sulphuric acid solution was connected to a pump which was calibrated at a flow rate of 17.5 ml/h. This sulphuric acid feeding rate corresponded with an estimated fumaric acid production rate during fermentation.

Crystallization was initiated by starting feeding the 0.8 M sulphuric acid solution to the vessel and stopped when the "fermentation" simulation time (50 h, pH 3) was fulfilled. The temperature was maintained at 30 °C.

After completion of the fermentation simulation, the fumaric acid crystals were recovered from the slurry which contained biomass, organic and inorganic acids by centrifugation at different G-forces for 1 minute (Figure 1, Table 2). Fumaric acid crystals were separated from the supernatant and analyzed for fumaric acid content by quantitative NMR. In addition, the nitrogen content was determined (Kjedahl-N) and the protein content was calculated (conversion factor 6.25, after substraction of ammonia-N).

The particle size (diameter) of the fumaric acid crystals formed in the fermentation simulation was measured using Malvern analysis (Malvern Mastersize S). The particle size (diameter) of the fumaric acid crystals ranged between 10 and 700 micrometres.

The results (Figure 1 and Table 2) show that it was possible to crystallize fumaric acid in the presence of microbial cells from a low pH medium. In addition it was shown that low centrifugation intensity (below 900 g x min) resulted in recovery of crude fumaric acid crystals with a purity of at least 84% w/w and a protein content of less than 10 g / kg fumaric acid crystals.

**Table 2: Total nitrogen content of fumaric acid crude crystals recovered by centrifugation at different G-forces for 1 minute.**

| G force (g) | Total N (mg/kg) | Total Protein (g/kg) |
|---|---|---|
| 300 | 839 | 5.2 |
| 900 | 1680 | 10.5 |
| 1500 | 2790 | 17.4 |
| 2000 | 3760 | 23.5 |
| 3000 | 5010 | 31.3 |

## Claims

1. Process for the crystallization of fumaric acid, comprising crystallizing fumaric acid in an aqueous solution having a pH of between 0.5 to 3.5, wherein the aqueous solution comprises microbial cells at a concentration of at least 0.5 to 20 g dry weight / I, wherein fumaric acid crystals are formed.

2. Process according to claim 1, wherein the aqueous solution is a fermentation broth.

3. Process according to claim 1 or 2, further comprising separating the fumaric acid crystals from the aqueous solution.

4. Process according to claim 3, wherein the separating comprises centrifugating the aqueous solution at low centrifugation intensity.

5. Process according to claim 4 wherein the centrifugation intensity is between 0.1 and 1000 x g. min.

6. Process according to any one of the claims 1 to 5 wherein the fumaric acid crystals comprise at least 80% w/w dry weight of fumaric acid.

7. Process according to any one of the claims 1 to 6, further comprising washing the fumaric acid crystals.

8. Fumaric acid crystals comprising 80% to 99.5 % w/w dry weight of fumaric acid and a protein content of between 0.1 and 15 g protein / kg fumaric acid.
